# EUROPEAN PATENT SPECIFICATION

(11) **EP 4 280 969 B1**
(45) Date of publication and mention of the grant of the patent: **03.12.2025**
(21) Application number: 21706107.6
(22) Date of filing: 25.01.2021
(51) Int. Cl.: A61B 10/02, A61B 18/02, A61B 18/00

(54) **BIOPSY SYSTEM AND DEVICE HAVING A CHILLED BIOPSY NEEDLE**
BIOPSIESYSTEM UND VORRICHTUNG MIT GEKÜHLTER BIOPSIENADEL
SYSTÈME ET DISPOSITIF DE BIOPSIE AYANT UNE AIGUILLE DE BIOPSIE REFROIDIE

(43) Date of publication of application: 29.11.2023
(73) Proprietor: Bard Peripheral Vascular, Inc., Franklin Lakes, NJ 07417 (US)
(72) Inventor: BOYLE, Brian, Franklin Lakes, NJ 07417 (US)
(74) Representative: Hoffmann Eitle
(86) International application number: PCT/US2021/014888
(87) International publication number: WO 2022/159112

(56) References cited:
- WO-A1-00/12010
- WO-A2-2008/023193
- US-A1- 2015 305 722

## Description

### Technical Field

The present invention relates to biopsy systems having biopsy needles, and, more particularly, to biopsy systems having biopsy needles that are coupled to a cooling element.

### Background Art

Percutaneous needle biopsy is standard of care for cancer diagnosis, because it allows for direct pathological assessment of suspected cancers. Pathological analysis is standard of care for cancer diagnosis, because it allows for visualization of the cellular structure of the tissue sample as well as chemical analysis of the tissue, including hormone receptors and genetic analysis.

Although biopsy is standard of care, biopsy also comes with complications and patient discomfort. For example, breast biopsy commonly includes complications such as bleeding, hematoma, and pain. Typically, these complications are managed with local anesthesia and epinephrine. Because biopsy requires tissue injury in order to access and sample the suspected tissue, some amount of bleeding is normal. However, the more these complications can be minimized, the better for the patient experience and the clinician experience.

Reduction of bleeding and pain are especially important in stereotactic and MRI biopsies, as well as fibroadenoma removals, where the procedures may take extended periods of time and where larger volumes of tissue may be removed. Reduction of bleeding will also reduce the time required to clean and disinfect equipment.

What is needed in the art is a biopsy device, biopsy system, and biopsy method that improve the patient experience and clinician experience by minimizing bleeding, hematoma, and pain during biopsy procedures.
WO 2008/023193 A2 discloses a medical device for sampling biological material, comprising: (i) an elongate housing; (ii) a probe for sampling the biological material slideably mounted within the elongate housing; and (iii) a current supply electrically coupled to the probe or the elongate housing; wherein said elongate housing and said probe are made from materials having different Peltier potentials such that supple of current from the current supply generates a Peltier effect between said elongate housing and said probe.

### Summary of Invention

The invention is defined by the appended independent claim. Further embodiments of the invention are illustrated in the dependent claims. The methods described herein do not form part of the invention, but are useful for understanding the invention.

### Summary of Disclosure

The present disclosure provides a biopsy system having a thermal cooling device. The biopsy system includes a biopsy device and a controller circuit. The biopsy device includes an elongate biopsy needle and a thermal cooling device. The elongate biopsy needle has a lumen, a proximal end portion, an intermediate portion, and a distal end portion. The elongate biopsy needle is made of a thermally conductive material.

Furthermore, the biopsy device includes a temperature transfer member that extends between the proximal end portion of the elongate biopsy needle and the thermal cooling device. The temperature transfer member has a first end and a second end.

The biopsy device includes a first thermo-mechanical coupler that is connected to the thermal cooling device and a second thermo-mechanical coupler that connects the second end of the temperature transfer member to the proximal end portion of the elongate biopsy needle.

The controller circuit is communicatively coupled to the thermal cooling device. The controller circuit is configured to control a chilling effect at the distal end portion of the elongate biopsy needle.

The disclosure in one form is directed to a biopsy device having an elongate biopsy needle, a thermal cooling device, and a temperature transfer member that extends between the elongate biopsy needle and the thermal cooling device. The elongate biopsy needle is chilled by the thermal cooling device.

More particularly, the elongate biopsy needle has a proximal end portion, an intermediate portion, and a distal end portion. The elongate biopsy needle is made of a thermally conductive material. The temperature transfer member extends between the proximal end portion of the elongate biopsy needle and the thermal cooling device. The temperature transfer member has a first end and a second end.

An advantage of the biopsy device is the inclusion of a first thermo-mechanical coupler that connects the first end of the temperature transfer member to the thermal cooling device. Moreover, the biopsy device includes a second thermo-mechanical coupler that connects the second end of the temperature transfer member to the proximal end portion of the elongate biopsy needle.

The disclosure in another form is directed to a biopsy device having a chilled biopsy needle or to be chilled biopsy needle. The biopsy device includes an elongate biopsy needle, a thermal cooling device, a thermal conductive mechanical member, a first thermo-mechanical coupler and a second thermo-mechanical coupler.

The elongate biopsy needle has a proximal end portion, an intermediate portion, and a distal end portion. The elongate biopsy needle is made of a thermally conductive material. The thermally conductive mechanical member has a first end and a second end.

An advantage of the present disclosure is that the thermally conductive mechanical member extends between the proximal end portion of the elongate biopsy needle and the thermal cooling device.

Another advantage is that the first thermo-mechanical coupler connects the first end of the thermally conductive mechanical member to the thermal cooling device. Yet another advantage is that a second thermo-mechanical coupler connects the second end of the thermally conductive mechanical member to the proximal end portion of the elongate biopsy needle.

### Brief Description of Drawings

The above-mentioned and other features and advantages of this disclosure, and the manner of attaining them, will become more apparent and the disclosure will be better understood by reference to the following description of embodiments taken in conjunction with the accompanying drawings, wherein:
Fig. 1 is a schematic representation of a biopsy system, including a schematic representation of a biopsy device;
Fig. 2 is a schematic representation of an exemplary embodiment of a biopsy system having a biopsy device; and
Fig. 3 is a schematic representation of an exemplary embodiment of a biopsy device.

Corresponding reference characters indicate corresponding parts throughout the several views. The exemplifications set out herein illustrate at least one embodiment of the invention, and such exemplifications are not to be construed as limiting the scope of the invention in any manner.

### Description of Embodiments

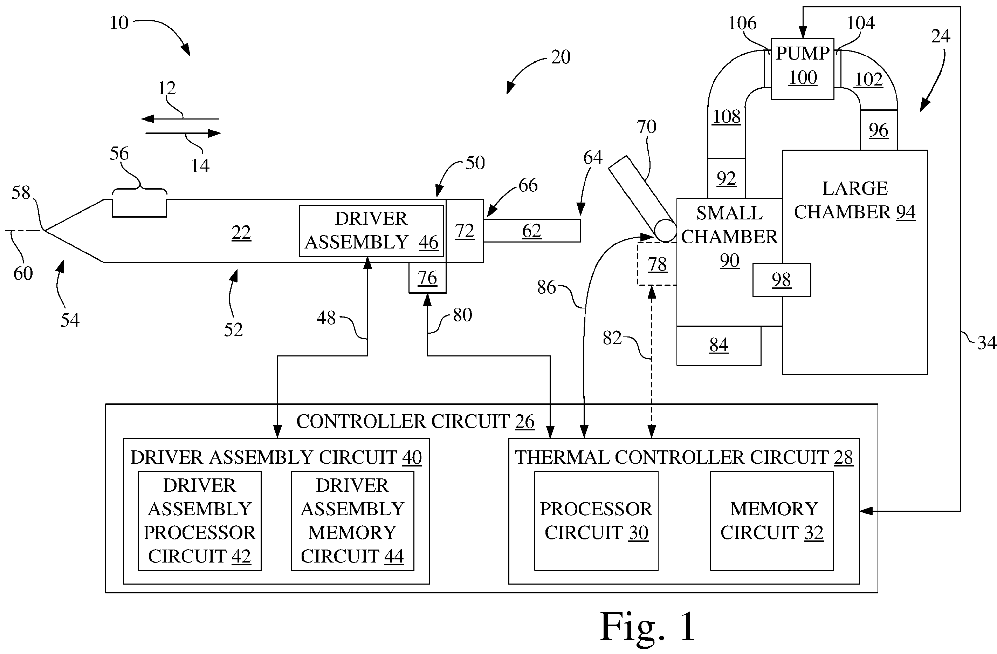

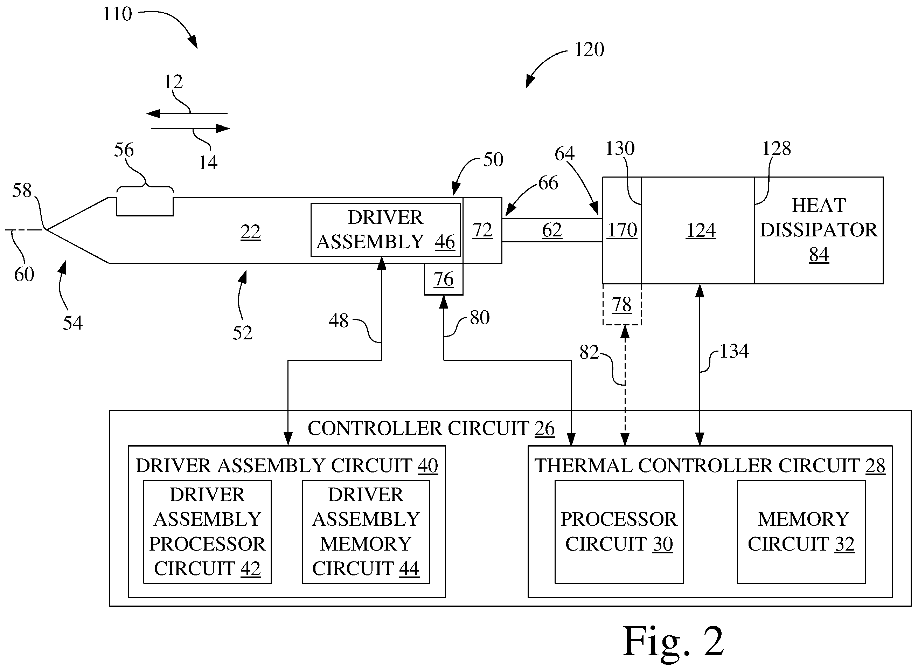

Referring now to the drawings, and more particularly to Fig. 1, there is shown a biopsy system 10 which generally includes a biopsy device 20, generally including an elongate biopsy needle 22 and a thermal cooling device 24, and a controller circuit 26. Controller circuit 26 is communicatively coupled to thermal cooling device 24. Elongate biopsy needle 22 is capable of being chilled or cooled. Controller circuit 26 is configured to control a chilling effect of elongate biopsy needle 22. To aid in the understanding of the elements, Figs. 1 and 2 include arrows showing distal direction 12 and proximal direction 14.

As shown in Figs. 1 and 2, controller circuit 26 includes a thermal controller circuit 28, which has a processor circuit 30 and memory circuit 32. Thermal controller circuit 28 is communicatively coupled to thermal cooling device 24 via communication link 34. Thermal controller circuit 28 may be assembled on an electrical circuit board. Processor circuit 30 has one or more programmable microprocessors and associated circuitry, such as input/output interface, digital thermometer, buffers, memory, etc. Memory circuit 32 is communicatively coupled to processor circuit 30, e.g., via a bus circuit, and is a non-transitory electronic memory that may include volatile memory circuits, such as random access memory (RAM), and non-volatile memory circuits, such as read only memory (ROM), electronically erasable programmable ROM (EEPROM), NOR flash memory, NAND flash memory, etc. Thermal controller circuit 28 may be formed as one or more Application Specific Integrated Circuits (ASIC).

Thermal controller circuit 28 is configured via software and/or firmware residing in memory circuit to execute program instructions to perform functions associated with the chilling effect generated by thermal cooling device 24, such as, controlling the operation of thermal cooling device 24, or, alternatively, controlling the operation of an electro-mechanical switch, as will be described further below in the description of the thermal cooling device 24 of Fig. 1.

In addition, as shown in Figs. 1 and 2, controller circuit 26 includes a driver assembly circuit 40. Driver assembly circuit 40 may be assembled on an electrical circuit board, and includes a driver assembly processor circuit 42 and a driver assembly memory circuit 44. Driver assembly processor circuit 42 has one or more programmable microprocessors and associated circuitry, such as input/output interface, clock, buffers, memory, etc. Driver assembly memory circuit 44 is communicatively coupled to driver assembly processor circuit 42, e.g., via a bus circuit, and is a non-transitory electronic memory that may include volatile memory circuits, such as random access memory (RAM), and non-volatile memory circuits, such as read only memory (ROM), electronically erasable programmable ROM (EEPROM), NOR flash memory, NAND flash memory, etc. Driver assembly circuit 40 may be formed as one or more Application Specific Integrated Circuits (ASIC).

As shown in Figs. 1 and 2, a driver assembly 46 is communicatively and controllably coupled to driver assembly circuit 40 via driver communication link 48, which may be wired or wireless. Driver assembly 46 is mechanically coupled to elongate biopsy needle 22. Driver assembly circuit 40 is configured via software and/or firmware residing in driver assembly memory circuit 44 to execute program instructions to perform functions associated with the retrieval of biopsy tissue samples, such as that of controlling the driver assembly 46 to engage elongate biopsy needle 22, such as, e.g., an outer cannula, an inner stylet, or both in translational movement, rotational movement, or both.

Each of the electrically powered elements of biopsy system 10 are electrically connected to an electrical power source, such as, a battery or, alternatively, each electrically powered element is configured to be connected to an AC wall power outlet to connect to the power grid.

Elongate biopsy needle 22 has a lumen (not shown in the schematic views of Figs. 1-3), a proximal end portion 50, an intermediate portion 52, and a distal end portion 54. Elongate biopsy needle 22 is made of a thermally conductive material, such as, e.g., aluminum, stainless steel, and nitinol.

Elongate biopsy needle 22 includes at least one of an elongate cannula and an elongate stylet. In some embodiments, elongate biopsy needle 22 includes an outer cutting cannula and an inner stylet. In other embodiments, elongate biopsy needle 22 includes an outer cannula and an inner cutting stylet. Distal end portion 54 includes a sample notch 56. Attached to or integral to distal end portion 54 is a piercing tip 58. Depending on the embodiment, piercing tip 58 is attached or integral to an outer cannula distal end portion of an outer cannula, or, alternatively, piercing tip 58 is attached or integral to a stylet distal end portion of an inner stylet.

Elongate biopsy needle 22 extends along a longitudinal axis 60. In order to distinguish intermediate portion 52 from proximal end portion 50, consider elongate biopsy needle 22 divided into thirds across longitudinal axis 60. Proximal end portion 50 is defined as the proximal-most third of elongate biopsy needle 22. Intermediate portion 52 is the middle third, and distal end portion 54 is the distal-most third of elongate biopsy needle 22.

Biopsy device 20 includes a temperature transfer member 62, which may also be described as a thermally conductive mechanical member. Temperature transfer member 62 extends between proximal end portion 50 of elongate biopsy needle 22 and thermal cooling device 24. Temperature transfer member 62 is made of a thermally conductive material, such as, e.g., aluminum, stainless steel, or copper, and is wrapped in insulation. As depicted schematically in Fig. 1, temperature transfer member 62 is a rigid, elongate solid cylinder of thermally conductive material, such as, metal, e.g., surgical grade stainless steel, copper, or aluminum. Temperature transfer member 62 has a first end 64 and a second end 66. First end 64 is proximal of second end 66.

Biopsy device 20 includes a first thermo-mechanical coupler 70 and a second thermo-mechanical coupler 72. Each of first thermo-mechanical coupler 70 and second thermo-mechanical coupler 72 are made of a thermally conductive material, such as, e.g., surgical grade stainless steel, copper, or aluminum. First thermo-mechanical coupler 70 is connected to thermal cooling device 24 and temperature transfer member 62. First thermo-mechanical coupler 70 is connected to thermal cooling device 24 to temperature transfer member 62. First thermo-mechanical coupler 70 is the connection between thermal cooling device 24 and first end 64 of temperature transfer member 62. First thermo-mechanical coupler 70 will be described in greater detail below.

Second thermo-mechanical coupler 72 is connected to second end 66 of temperature transfer member 62. Second thermo-mechanical coupler 72 connects second end 66 of temperature transfer member 62 to proximal end portion 50 of elongate biopsy needle 22. Second thermo-mechanical coupler 72 is the connection between temperature transfer member 62 and proximal end portion 50 of elongate biopsy needle 22. Once biopsy device 20 is fully assembled, second thermo-mechanical coupler 72 is releasably connected to proximal end portion 50 of elongate biopsy needle 22 and temperature transfer member 62. For example, second thermo-mechanical coupler 72 may take the form of a pin vise or collet. The more surface area that second thermo-mechanical coupler 72 has in contact with proximal end portion 50 of elongate biopsy needle 22, the more heat will transfer from distal end portion 54 of elongate biopsy needle 22 through the intermediate portion to proximal end portion 50 of elongate biopsy needle 22 and to second thermo-mechanical coupler 72, which, in turn, is cooled by thermal cooling device 24 of biopsy device 20 of Fig. 1 through temperature transfer member 62 and first thermo-mechanical coupler 70.

As shown in Fig. 1, thermal cooling device 24 is in the form of a dual chamber enclosure that has a first smaller chamber 90 having a first valve 92 and a second larger chamber 94 having a second valve 96. The first chamber 90 is smaller in volume than the second chamber 94. First smaller chamber 90 and second larger chamber 94 are fluidly connected through a third valve 98. In addition, thermal cooling device 24 includes a pump 100 having two ports, a first port 104 and a second port 106, and a first conduit 102 and a second conduit 108. Pump 100 is coupled to each of first valve 92 and second valve 96. Each of first valve 92, second valve 96, and third valve 98 is one-directional. For example, according to thermal cooling device 24 of Fig. 1, pump 100 is configured to pump fluid only in the direction from second larger chamber 94 to pump 100 via negative pressure, and, then, from pump 100 to first smaller chamber 90 by applying positive pressure. Thus, according to the drawing of Fig. 1, the fluid is moved in a counterclockwise direction, but, of course, the components of thermal cooling device 24 of Fig. 1 may be reorganized to operate in a clockwise pattern if desired. The biopsy system 10 of Fig. 1 includes a heat dissipator 84, e.g., a heat sink, a radiator element, or a venting mechanism, to dissipate heat away from the biopsy system.

Thermal cooling device 24 of Fig. 1 has a fixed amount of gas, where the amount is expressed in moles (symbol - mol). Thermal cooling device 24 has two modes of operation, a first mode of operation, which produces an exothermic effect, i.e., heat energy is produced, and a second mode of operation, which produces an endothermic effect, i.e., a chilling effect is produced.

According to the embodiment of Fig. 1, thermal cooling device 24 is configured to engage in the first mode of operation during a first period of engagement, and thermal cooling device 24 is configured to be engaged in the second mode of operation during a second period of engagement. Thermal cooling device 24 is configured to close third valve 98 during the first period of engagement, and thermal cooling device 24 is configured to open third valve 98 during the second period of engagement. Third valve 98 is configured to be in a sealed state during a first period of engagement, and third valve 98 is configured to be in an open state during a second period of engagement.

Thermal cooling device 24 switches between the two modes of operation. Biopsy system 10 of Fig. 1, in particular, thermal controller circuit 28 is configured to switch thermal cooling device 24, in particular pump 100, off and on during a medical procedure. Alternatively, biopsy system 10 of Fig. 1 is configured to continuously operate thermal cooling device 24 during a medical procedure. The first period of engagement and the second period of engagement are cyclically repeated during the operation of biopsy device 20.

During the first period of engagement, thermal cooling device 24 is configured to generate an exothermic process by using pump 100 to compress gas into first smaller chamber 90 from second larger chamber 94. More specifically, pump 100 is first used to generate a vacuum in first conduit 102, which suctions gas from second larger chamber 94 via second valve 96 into the first conduit 102 via first port 104 in pump 100 and into pump 100. Pump 100 is configured to pump fluid from pump 100 out second port 106 into second conduit 108 and into first smaller chamber 90, compressing the fixed amount of gas inside first smaller chamber 90. Heat dissipator 84 (e.g., a heat sink, a radiator element, or a venting mechanism, such as, a fan) is connected to first smaller chamber 90 to aid in the removal of heat energy from first smaller chamber 90 generated during the first period of engagement.

During the second period of engagement, thermal cooling device 24 is configured to generate an endothermic process, which creates a chilling effect, by releasing the compressed gas from first smaller chamber 90 into second larger chamber 94 by opening third valve 98. During the second period of engagement, pump 100 may be on or off, depending on the desired chilling effect.

In Fig. 1, first thermo-mechanical coupler 70 is in the form of an electro-mechanical switch configured to be selectively opened to thermally isolate thermal cooling device 24 from elongate biopsy needle 22 and to be selectively closed to transfer a chilling effect generated by thermal cooling device 24 to temperature transfer member 62 and ultimately to distal end portion 54 of elongate biopsy needle 22. Once the chilled effect reaches distal end portion 54 of elongate biopsy needle 22, the elongate biopsy needle may be referred to herein as a chilled needle. Controller circuit 26, in particular, thermal controller circuit 28 is communicatively coupled to first thermo-mechanical coupler 70 via thermo-mechanical coupler communication link 86, which may be wired or wireless. Controller circuit 26, in particular, thermal controller circuit 28, is configured to selectively connect first thermo-mechanical coupler 70 to first end 64 of temperature transfer member 62. Thermal controller circuit 28 is configured to open first thermo-mechanical coupler 70 during the first period of engagement, when thermal cooling device 24 is generating heat energy, by generating and delivering an open instruction to first thermo-mechanical coupler 70 via thermo-mechanical coupler communication link 86. In the open condition, first thermo-mechanical coupler 70 thermally isolates elongate biopsy needle 22 from thermal cooling device 24, so that elongate biopsy needle 22 does not receive heat generated as a by-product of the first period of engagement.

Furthermore, thermal controller circuit 28 is configured to generate and deliver a close instruction to first thermo-mechanical coupler 70 via thermo-mechanical coupler communication link 86. Thermal controller circuit 28 is configured to close first thermo-mechanical coupler 70 during the second period of engagement, when thermal cooling device 24 is generating a chilling effect. As a result of receiving the close instruction, first thermo-mechanical coupler 70 moves into a closed condition during the second period of engagement of thermal cooling device 24. The closed condition of first thermo-mechanical coupler 70 completes a thermally conductive connection between thermal cooling device 24 and first end 64 of temperature transfer member 62.

Preferably, but not necessarily, biopsy system 10 of Fig. 1 includes at least one thermal sensor. For example, as shown in Fig. 1, a first thermal sensor 76 is shown coupled to proximal end portion 50 of elongate biopsy needle 22. Alternatively, as shown in Fig. 1, first thermal sensor 76 may be replaced with a second thermal sensor 78 placed directly in contact with and in direct connection with first thermo-mechanical coupler 70. It is also contemplated that biopsy system 10 may include both second thermal sensor 78 positioned adjacent to first thermo-mechanical coupler 70 and first thermal sensor 76 present at proximal end portion 50 of elongate biopsy needle 22.

Thermal controller circuit 28 is communicatively coupled to first thermal sensor 76, if present, via a first thermal sensor communication link 80, which may be wired or wireless. Additionally or alternatively, thermal controller circuit 28 is communicatively coupled to second thermal sensor 78, if present, via a second thermal sensor communication link 82, which may be wired or wireless. First thermal sensor 76 is configured to sense at least one temperature datum of proximal end portion 50 of elongate biopsy needle 22, and first thermal sensor 76 is configured deliver the at least one temperature data to thermal controller circuit 28. Second thermal sensor 78 is configured to sense at least one temperature datum of first thermo-mechanical coupler 70 and second thermal sensor 78 is configured to deliver the at least one temperature datum to thermal controller circuit 28. Once thermal controller circuit 28 receives the at least one temperature datum, it is referred to as received temperature data. Thermal controller circuit 28 is configured to compare received temperature data to a threshold temperature setting stored in memory circuit 32.

According to an exemplary embodiment, if any of the received temperature data is above the threshold temperature setting, then thermal controller circuit 28 processes a set of thermal cooling instructions in the processor circuit 30 to selectively engage thermal cooling device 24 to generate a chilling effect. On the other hand, if thermal controller circuit 28 determines that all of the received temperature data is at or below the threshold temperature setting, then thermal controller circuit 28 continues to sense all temperature data received from the at least one thermal sensor, such as, e.g., first thermal sensor 76 and second thermal sensor 78, and thermal cooling device 24 is not engaged to generate a chilling effect. Moreover, according to this exemplary embodiment, when the received temperature data is at or below the threshold temperature setting, thermal controller circuit 28 may control pump 100 to engage in the first period of engagement, thereby preparing the thermal cooling device 24 for entry into the second period of engagement at the moment the received temperature data is detected to be above the threshold temperature setting. Once thermal controller circuit 28 determines that the received temperature data is above the threshold temperature setting, then thermal controller circuit 28 communicates the set of thermal cooling instructions to thermal cooling device 24 to enter the second period of engagement, which produces a chilling effect. According to this exemplary embodiment of biopsy system 10 of Fig. 1, thermal cooling device 24 is not continuously operated in a loop from first period of engagement to second period of engagement and back again on a regular cycle, but instead thermal cooling device 24 enters the first period of engagement, during which gas is compressed inside first smaller chamber 90, as a result of thermal controller circuit 28 determining that the received temperature data is at or below the threshold temperature setting. The chilling effect of biopsy system 10 of Fig. 1 may be regulated by a feedback loop through processor circuit 30 and at least one thermal sensor, such as, e.g., first thermal sensor 76 and second thermal sensor 78. The chilling effect may be automatically generated or manually controlled as part of a handheld device or a console capital component. Thermal controller circuit 28 is configured to manipulate thermal cooling device 24 to lengthen or shorten the second period of engagement depending on the received temperature data received by first thermal sensor 76 and/or second thermal sensor 78.

Additionally or alternatively, if any of the received temperature data from first thermal sensor 76 and/or second thermal sensor 78 is above the threshold temperature setting, then thermal controller circuit 28 is configured to deliver the close instruction to the first thermo-mechanical coupler 70, provided that thermal cooling device 24 is currently engaged in the second period of engagement, which produces a chilling effect.

In an exemplary embodiment, if received temperature data from first thermal sensor 76 is higher than received temperature data from second thermal sensor 78, thermal controller circuit 28 is configured to control first thermo-mechanical coupler 70 to be in the closed condition. Moreover, if received temperature data from first thermal sensor 76 is lower than received temperature data from second thermal sensor 78, thermal controller circuit 28 is configured to control first thermo-mechanical coupler 70 to be in the open condition.

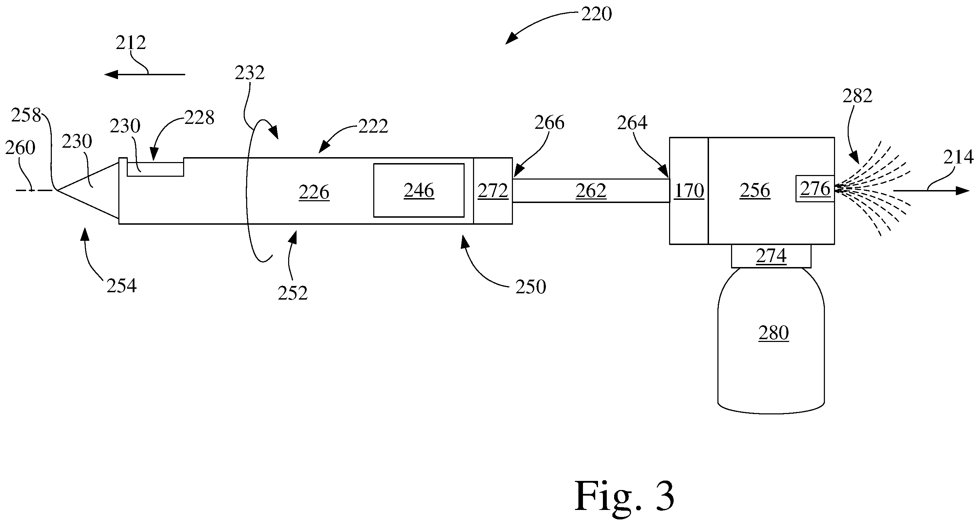

As shown in Figs. 2 and 3, an alternative first thermo-mechanical coupler 170 replaces the first thermo-mechanical coupler 70 of biopsy device 20 of Fig. 1. Alternative first thermo-mechanical coupler 170 is not an electro-mechanical switch, but instead may take the form of a pin vise or collet. In the second biopsy system 110 and second biopsy device 120 of Fig. 2, alternative first thermo-mechanical coupler 170 fixedly connects a second thermal cooling device 124 to first end 64 of temperature transfer member 62 once second biopsy device 120 is in the fully assembled state.

Fig. 2 shows second biopsy system 110 having a second biopsy device 120 that includes elongate biopsy needle 22, second thermal cooling device 124, temperature transfer member 62, alternative first thermo-mechanical coupler 170 and second thermo-mechanical coupler 72. Elongate biopsy needle 22, temperature transfer member 62, and second thermo-mechanical coupler 72 are the same as those described above with reference to biopsy system 10 of Fig. 1. Moreover, controller circuit 26 of Fig. 1 also applies to controller circuit 26 of Fig. 2, and, thus, the reference numerals will remain the same. Controller circuit 26, specifically thermal controller circuit 28 is communicatively linked to the second thermal cooling device 124 via second communication link 134.

As shown in Fig. 2, second thermal cooling device 124 is in the form of a thermoelectric heat pump, such as, e.g., a Peltier heat pump, which, during operation, includes a hot side 128 and a cold side 130. For example, the differential in temperature between hot side 128 and cold side 130 is up to and including 70°C. The chilling effect is transferred through the thermally conductive materials of the elements of the second biopsy device 120 from cold side 130 of second thermal cooling device 124 to distal end portion 54 of elongate biopsy needle 22. Optionally, second biopsy system 110 includes heat dissipator 84, e.g., a heat sink, a radiator element, or a venting mechanism, coupled to hot side 128 of second thermal cooling device 124. Additionally or alternatively, heat dissipator 84 may be coupled to proximal end portion 50 of elongate biopsy needle 22.

Third biopsy device 220, having a chilled biopsy needle, is shown in Fig. 3. Third biopsy device 220 may replace biopsy device 20 in biopsy system 10 of Fig. 1. Third biopsy device 220 may replace biopsy device 120 in second biopsy system 110 of Fig. 2.

As shown in Fig. 3, the third elongate biopsy needle 222 includes an outer elongate cannula 226, having a sample port 228, and an inner elongate stylet 230. The inner elongate stylet 230 is configured to rotate in a first rotational direction 232, as shown in Fig. 3, a second rotational direction, or both, as directed by a third driver assembly 246. Third elongate biopsy needle 222 extends along third longitudinal axis 260.

Third biopsy device 220 includes third elongate biopsy needle 222 having a third elongate biopsy needle proximal end portion 250, a third elongate biopsy needle intermediate portion 252, and a third elongate biopsy needle distal end portion 254. Third elongate biopsy needle 222 is made of a thermally conductive material, such as, e.g., aluminum, stainless steel, or copper. Fig. 3 shows inner elongate stylet 230 includes a third piercing tip 258. However, in other embodiments, outer elongate cannula 226 may include a piercing tip.

As shown in Fig. 3, third biopsy device 220 includes a third thermal cooling device 256. Third biopsy device 220 includes a thermally conductive mechanical member 262 that extends between third elongate biopsy needle proximal end portion 250 of third elongate biopsy needle 222 and third thermal cooling device 256. Thermally conductive mechanical member 262 has a thermally conductive mechanical member first end 264 and a thermally conductive mechanical member second end 266. Thermally conductive mechanical member 262 is a rigid, elongate solid cylinder comprised of metal, such as, e.g., aluminum, stainless steel, or copper. Alternative first thermo-mechanical coupler 170 directly and fixedly connects thermally conductive mechanical member first end 264 to third thermal cooling device 256, when third thermal cooling device 256 is fully assembled. An alternative second thermo-mechanical coupler 272 connects thermally conductive mechanical member second end 266 to third elongate biopsy needle proximal end portion 250 of third elongate biopsy needle 222. Alternative second thermo-mechanical coupler 272 may take the form of a pin vise or collet. The more surface area that alternative second thermo-mechanical coupler 272 has in contact with third elongate biopsy needle proximal end portion 250 of third elongate biopsy needle 222, the more heat will transfer from third elongate biopsy needle 222 to alternative second thermo-mechanical coupler 272.

Third thermal cooling device 256 includes a canister valve 274 and an ambient air port 276. Third thermal cooling device 256 is configured to couple with alternative first thermo-mechanical coupler 170. Canister valve 274 is configured to receive a disposable canister 280 of compressed gas, such as, e.g. liquid Nitrogen, which is commonly available off-the-shelf. Third thermal cooling device 256 is configured to open the canister valve 274 in order to release the compressed gas from disposable canister 280 to the ambient air through ambient air port 276 as shown in Fig. 3 as gas release 282. Alternatively, the ambient air port 276 may be coupled to an exhaust vent (not shown) or to a larger container (not shown). As the compressed gas escapes disposable canister 280, a chilling effect is generated by third thermal cooling device 256. As a result, heat energy present in third elongate biopsy needle distal end portion 254 travels in a proximal direction 14 toward the chilling effect. The chilling effect is thermally transferred through the thermally conductive material of third thermal cooling device 256 to alternative first thermo-mechanical coupler 170 and then to thermally conductive mechanical member first end 264 through thermally conductive mechanical member 262 to thermally conductive mechanical member second end 266. The chilling effect is thermally transferred from thermally conductive mechanical member second end 266 to alternative second thermo-mechanical coupler 272 and on to third elongate biopsy needle proximal end portion 250 and onward distally through third elongate biopsy needle intermediate portion 252 to third elongate biopsy needle distal end portion 254. Third thermal cooling device 256 does not cyclically repeat the generation of the chilling effect. Third thermal cooling device 256 may repeat the generation of the chilling effect only after first uncoupling the emptied canister, attaching a disposable canister 280 filled with compressed gas, and repeating the process of releasing the compressed gas through canister valve 274 and ambient air port 276. Third thermal cooling device 256 may be operated manually or through a biopsy system, such as, e.g., second biopsy system 110 of Fig. 2.

The disclosure, in another form thereof, is directed to a method for chilling elongate biopsy needle 22 during a biopsy procedure. The method includes penetrating a distal end portion 54 of elongate biopsy needle 22 of a biopsy device 20 into a patient. The method further includes continuing to advance the distal end portion 54 of elongate biopsy needle 22 until the distal end portion 54 reaches the target tissue. In some embodiments, the method further includes collecting a tissue sample from the target tissue in the patient and retracting the tissue sample through to the proximal end portion 50 of biopsy device 20. The method includes collecting and monitoring one or more temperature readings taken at a proximal end portion 50 of elongate biopsy needle 22. The one or more temperature readings are taken by a first thermal sensor 76 positioned on or adjacent to proximal end portion 50 of elongate biopsy needle 22. The one or more temperature readings are communicated to a thermal controller circuit 28 through a first thermal sensor communication link 80. As described above, thermal controller circuit 28 includes memory circuit 32 and processor circuit 30. If any of the temperature readings taken at proximal end portion 50 of elongate biopsy needle 22 is higher than a set maximum temperature stored in memory circuit 32, then thermal cooling device 24 will be activated to create a chilling effect at proximal end portion 50 of elongate biopsy needle 22.

The chilling effect generated by thermal cooling device 24 is transferred to proximal end portion 50 of elongate biopsy needle 22 first through first thermo-mechanical coupler 70 that directly connects a first end 64 of a temperature transfer member 62 to thermal cooling device 24. Then, the chilling effect is conveyed in a distal direction 12 through temperature transfer member 62 to a second end of temperature transfer member 62. The second end of temperature transfer member 62 is connected to second thermo-mechanical coupler 72, which is directly connected to proximal end portion 50 of elongate biopsy needle 22. Thus, the chilling effect generated by thermal cooling device 24 is transferred from thermal cooling device 24 through first thermo-mechanical coupler 70 to the first end of temperature transfer member 62 in a distal direction 12 across temperature transfer member 62 to the second end of temperature transfer member 62 and from second thermo-mechanical coupler 72 ultimately to proximal end portion 50 of elongate biopsy needle 22. Since elongate biopsy needle 22 is composed of a thermally conductive material, the chilling effect is transferred from proximal end portion 50 to distal end portion 54 of elongate biopsy needle 22, including piercing tip 58. Naturally, because energy moves from hot to cold to achieve equilibrium, heat energy present in distal end portion 54 of elongate biopsy needle 22 is conducted in the proximal direction 14 toward thermal cooling device 24 when thermal cooling device 24 is cooler than the temperature of the distal end portion 54 of elongate biopsy needle 22 during a biopsy procedure. Heat from distal end portion 54 of elongate biopsy needle 22 is conducted along elongate biopsy needle 22 in a proximal direction 14 to second thermo-mechanical coupler 72, then from second thermo-mechanical coupler 72 to second end 66 of the temperature transfer member 62, along temperature transfer member 62 to first end 64 of temperature transfer member 62, from first end 64 to first thermo-mechanical coupler 70, and from first thermo-mechanical coupler 70 to thermal cooling device 24 where the remaining heat energy is dissipated out through convection to the ambient air. Moreover, thermal cooling device 24 may have an additional heat dissipator 84 e.g., a heat sink, a radiator element, or a venting mechanism, connected to it for the remainder of the heat energy to dissipate. Alternatively, thermal cooling device 24 may further include a second heat dissipator, such as, an exhaust fan to further exhaust the dissipated heat into the room in which the biopsy system 10 resides.

Advantageously, cooling elongate biopsy needle 22 during a biopsy procedure reduces the need for post procedure compression and helps to expedite overall procedure time. The chilling effect reduces bleeding and pain. Reduction in the sensation of pain may reduce the need for anesthetic and epinephrine. Reduction of bleeding is especially advantageous in stereotactic and MRI biopsies, as well as fibroadenoma removals, where procedures may take extended periods of time and where larger volumes of tissue may be removed. Cooling elongate biopsy needle 22 also reduces external bleeding, as the blood in contact with the outer cannula is more viscous and less likely to travel via capillary action over the outer cannula and out the access site. Reduction in bleeding also reduces the time required to clean and disinfect equipment. Furthermore, because the chilled effect extends to the tissue itself, and because chilled tissue cuts more cleanly than warmed tissue, biopsy sample quality is improved by the disclosed biopsy systems, biopsy devices, and methods. Thus, the disclosed biopsy systems, biopsy devices, and methods improve diagnostic outcomes by improving tissue sample quality.

The following items also relate to the disclosure:

In one embodiment, the disclosure relates to a biopsy system. The biopsy system may include a biopsy device, a thermal cooling device for thermal cooling (of a biopsy needle of the biopsy device), a temperature transfer member, a first thermo-mechanical coupler, a second thermo-mechanical coupler, and a controller circuit. The biopsy device may have an elongate biopsy needle. In accordance with any of the embodiments, the elongate biopsy needle may have a lumen, a proximal end portion, an intermediate portion, and a distal end portion. The elongate biopsy needle may be made of a thermally conductive material. The temperature transfer member may extend between the proximal end portion of the elongate biopsy needle and the thermal cooling device. The temperature transfer member may have a first end and a second end. The first thermo-mechanical coupler may be connected or configured to connect to the thermal cooling device. The second thermo-mechanical coupler connects or may be configured to connect the second end of the temperature transfer member to the proximal end portion of the elongate biopsy needle. The controller circuit may be communicatively coupled or may be configured to communicatively couple to the thermal cooling device. The controller circuit may control or may be configured to control a chilling (cooling) effect at the distal end portion of the elongate biopsy needle.

In accordance with the embodiment, the biopsy device may include a driver assembly that may move or may be configured to move the elongate biopsy needle. The controller circuit may include a driver assembly circuit and a thermal controller circuit. The driver assembly circuit may be communicatively coupled or may be configured to be communicatively coupled to the driver assembly. The thermal controller circuit may be communicatively coupled or may be configured to be communicatively coupled to the thermal cooling device. This may allow for the thermal controller circuit to deliver communications, e.g., signals to open and/or signals to close, to an electro-mechanical switch in order to control the opening and closing of the electro-mechanical switch; the thermal controller circuit to deliver a set of thermal cooling instructions to the thermal cooling device to enter a second period of engagement, which produces a chilling effect at the elongate biopsy needle; and/or the thermal controller circuit to deliver communication signals to the thermal cooling device to regulate the chilling effect of the biopsy device via a continuously operated feedback loop through a processor circuit and at least one thermal sensor.

In accordance with any of the embodiments, the controller circuit may selectively connect or may be configured to selectively connect the first thermo-mechanical coupler to the first end of the temperature transfer member. This may allow for the thermal controller circuit to deliver signals to open and signals to close to the electro-mechanical switch in order to control the opening and closing of the electro-mechanical switch, respectively.

In accordance with any of the embodiments of the biopsy system, the first thermo-mechanical coupler may be an electro-mechanical switch that may selectively connect or may be configured to selectively connect the thermal cooling device to the first end of the temperature transfer member. The thermal controller circuit may open or may be configured to open the electro-mechanical switch during a first period of engagement. The thermal cooling device may produce or may be configured to produce an exothermic effect during the first period of engagement.

In accordance with any of the embodiments, the first thermo-mechanical coupler may be an electro-mechanical switch that may selectively connect or may be configured to selectively connect the thermal cooling device to the first end of the temperature transfer member. The thermal control circuit may close or may be configured to close the electro-mechanical switch during a second period of engagement. The thermal cooling device may produce or may be configured to produce an endothermic effect during the second period of engagement.

In accordance with any of the embodiments, the biopsy system may include a thermal sensor that may be coupled or configured to be coupled to the proximal end portion of the elongate biopsy needle. The controller circuit may include a thermal controller circuit. The thermal controller circuit may include a processor circuit and a memory circuit. The thermal controller circuit may be communicatively coupled or may be configured to be communicatively coupled to the thermal sensor. The thermal sensor may collect and deliver or may be configured to collect and deliver temperature datum to the thermal control circuit. The thermal controller circuit may be communicatively coupled or configured to be communicatively coupled to the thermal cooling device. The thermal controller circuit may compare or may be configured to compare the temperature datum to a threshold stored in the memory circuit, and if the temperature datum is above the threshold then the thermal controller circuit processes or is configured to process a thermal cooling instruction in the processor circuit to selectively engage the thermal cooling device to generate a chilling effect. If the thermal controller circuit determines that the temperature datum is at or below the threshold, then the thermal controller circuit continues to sense all temperature data received from the thermal sensor and the thermal cooling device is not engaged.

In accordance with some of the embodiments, the biopsy system may continuously operate or may be configured to continuously operate the thermal cooling device during a medical procedure.

In accordance with some of the embodiments of the biopsy system, the thermal cooling device may include a dual chamber enclosure and a pump. The dual chamber enclosure includes a first (smaller) chamber and a (second) larger chamber, wherein the first chamber may be smaller than the second chamber. The first smaller chamber may have a first valve, and the second larger chamber may have a second valve. The first smaller chamber and the second larger chamber may be fluidly connected or may be configured for fluid connection through a third valve. The third valve may be seal or opened, or configured to be sealed or opened. The pump may be coupled or configured to be coupled to each of the first valve and the second valve. The thermal cooling device may generate or may be configured to generate an exothermic process by using the pump to compress gas into the first smaller chamber from the second larger chamber. The thermal cooling device may generate or may be configured to generate an endothermic process by releasing the gas from the first smaller chamber into the second larger chamber by opening the third valve. The controller circuit may be communicatively coupled or configured to be communicatively coupled to the pump.

In another embodiment, the disclosure relates to a biopsy device that includes a chilled or to be chilled (chillable) biopsy needle. The biopsy device may be a biopsy device of any of the preceding paragraphs and may be comprised in the biopsy system of any of the preceding paragraphs. The biopsy device may include an elongate biopsy needle, a thermal cooling device, a temperature transfer member, a first thermo-mechanical coupler, and a second thermo-mechanical coupler. The elongate biopsy needle may have a proximal end portion, an intermediate portion, and a distal end portion. The elongate biopsy needle may be made of a thermally conductive material. The temperature transfer member may extend between the proximal end portion of the elongate biopsy needle and the thermal cooling device. The temperature transfer member may have a first end and a second end. The first thermo-mechanical coupler may connect or may be configured to connect the first end of the temperature transfer member to the thermal cooling device. The second thermo-mechanical coupler may connect or may be configured to connect the second end of the temperature transfer member to the proximal end portion of the elongate biopsy needle.

In accordance with any of the embodiments of the biopsy device, the elongate biopsy needle may include at least one of an elongate cannula and an elongate stylet. The temperature transfer member may be a rigid, elongate solid cylinder that may be made of metal.

In accordance with some of the embodiments of the biopsy device, the thermal cooling device may be a thermoelectric heat pump.

In accordance with some of the embodiments of the biopsy device, the thermal cooling device may include a canister valve and an ambient air port. The thermal cooling device may be coupled or may be configured to couple with the first thermo-mechanical coupler. The canister valve may receive or may be configured to receive a disposable canister containing compressed gas. The thermal cooling device may open or may be configured to open the canister valve in order to release the compressed gas from the disposable canister to the ambient air through the ambient air port to generate a chilling effect that may be transferred from the thermal cooling device to the first thermo-mechanical coupler, to the temperature transfer member, to the proximal end portion, and to the distal end portion of the elongate biopsy needle.

In accordance with some of the embodiments of the biopsy device, the thermal cooling device may include a dual chamber enclosure and a pump. The dual chamber enclosure may include a first (smaller) chamber and a second (larger) chamber, wherein the first chamber may be smaller than the second chamber. The first chamber may have a first valve, and the second larger chamber may have a second valve. The first smaller chamber and the second larger chamber may be fluidly coupled or may be configured for fluid coupling through a third valve. The third valve may be sealed or opened or may be configured to be sealed or opened. The pump may be coupled to each of the first valve and the second valve. The thermal cooling device may generate or may be configured to generate an exothermic process by using the pump to compress gas into the first smaller chamber from the second larger chamber. The thermal cooling device may generate or may be configured to generate an endothermic process by releasing the gas from the first smaller chamber into the second larger chamber by opening the third valve.

In accordance with the embodiment of the thermal cooling device having a dual chamber enclosure or the embodiment of the thermal cooling device that may be a thermoelectric heat pump, the thermal cooling device may include a first thermal sensor coupled or configured to be coupled to the proximal end portion of the elongate biopsy needle. The first thermal sensor may sense or may be configured to sense the temperature of the proximal end portion of the elongate biopsy needle.

In accordance with either embodiment of the thermal cooling device of the preceding paragraph, the thermal cooling device may include a second thermal sensor coupled or configured to be to the first thermo-mechanical coupler. The thermal sensor may sense or may be configured to sense the temperature of the first thermo-mechanical coupler.

In another embodiment, the disclosure relates to a biopsy device that includes a chilled or to be chilled (chillable) biopsy needle. The biopsy device may a be biopsy device of any of the preceding paragraphs and may be comprised in the biopsy system of any of the preceding paragraphs. The biopsy device may include an elongate biopsy needle, a thermal cooling device, a thermally conductive mechanical member, a first thermo-mechanical coupler, and a second thermo-mechanical coupler. The elongate biopsy needle may have a proximal end portion, an intermediate portion, and a distal end portion. The elongate biopsy needle may be made of a thermally conductive material. The thermally conductive mechanical member may extend between the proximal end portion of the elongate biopsy needle and the thermal cooling device. The thermally conductive mechanical member may have a first end and a second end. The first thermo-mechanical coupler may connect or may be configured to connect the first end of the thermally conductive mechanical member to the thermal cooling device. The second thermo-mechanical coupler may connect or may be configured to connect the second end of the thermally conductive mechanical member to the proximal end portion of the elongate biopsy needle.

In accordance with any of the embodiments of the biopsy device, the elongate biopsy needle may include at least one of an elongate cannula and an elongate stylet, and the thermally conductive mechanical member may be a rigid, elongate solid cylinder that may be made of metal.

Optionally, in accordance with some embodiments, the thermal cooling device may be a thermoelectric heat pump.

In accordance with some embodiments, the first thermo-mechanical coupler may be an electro-mechanical switch, and the electro-mechanical switch may open or may be configured to open during a first period of engagement of the thermal cooling device to thermally isolate the elongate biopsy needle from the thermal cooling device.

In accordance with some embodiments, the electro-mechanical switch may move or may be configured to move into a closed condition during a second period of engagement of the thermal cooling device. The closed condition completes a thermal connection between the thermal cooling device and the first end of the thermally conductive mechanical member.

The biopsy device of the preceding paragraphs [0075] to [0079] may be characterized by the features of the biopsy device and the remaining entities of any of the preceding paragraphs [0060] to [0074].

While this invention has been described with respect to at least one embodiment, the present invention can be further modified within the scope of this disclosure This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A biopsy device (20, 120, 220) having a biopsy needle (22, 222) to be chilled, comprising:
an elongate biopsy needle (22, 222) having a proximal end portion (50, 250), an intermediate portion (52, 252), and a distal end portion (54, 254), the elongate biopsy needle (22, 222) being made of a thermally conductive material;
a thermal cooling device (24, 124, 256);
a temperature transfer member (62, 262) that extends between the proximal end portion (50, 250) of the elongate biopsy needle (22, 222) and the thermal cooling device (24, 124, 256), the temperature transfer member (62, 262) having a first end (64, 264) and a second end (66, 266);
a first thermo-mechanical coupler (70, 170) that connects the first end (64, 264) of the temperature transfer member (62, 262) to the thermal cooling device (24, 124, 256); and
a second thermo-mechanical coupler (72, 272) that connects the second end (66, 266) of the temperature transfer member (62, 262) to the proximal end portion (50, 250) of the elongate biopsy needle (22, 222).

2. The biopsy device (20, 120, 220) according to claim 1, wherein the elongate biopsy needle (22, 222) includes at least one of an elongate cannula (226) and an elongate stylet (230), and the temperature transfer member (62, 262) is a rigid, elongate solid cylinder comprised of metal.

3. The biopsy device (20, 120, 220) according to any one of claims 1 to 2, wherein the thermal cooling device (24, 124, 256) is a thermoelectric heat pump.

4. The biopsy device (20, 120, 220) according to any one of claims 1 to 2, wherein the thermal cooling device (24, 124, 256) comprises a canister valve (274) and an ambient air port (276), the thermal cooling device (24, 124, 256) being configured to couple with the first thermo-mechanical coupler (70, 170), and the canister valve (274) configured to receive a disposable canister (280) containing compressed gas, wherein the thermal cooling device (24, 124, 256) is configured to open the canister valve (274) in order to release the compressed gas from the disposable canister (280) to the ambient air through the ambient air port (276) to generate a chilling effect that is transferred from the thermal cooling device (24, 124, 256) to the first thermo-mechanical coupler (70, 170), to the temperature transfer member (62, 262), to the proximal end portion (50, 250), and to the distal end portion (54, 254) of the elongate biopsy needle (22, 222).

5. The biopsy device (20, 120, 220) according to any one of claims 1 to 2, wherein the thermal cooling device (24, 124, 256) comprises:
a dual chamber enclosure, the dual chamber enclosure comprises a first smaller chamber (90) having a first valve (92) and a second larger chamber (94) having a second valve (96), the first smaller chamber (90) and the second larger chamber (94) are fluidly coupled through a third valve (98) configured to be sealed or opened; and
a pump coupled to each of the first valve (92) and the second valve (96),
wherein the thermal cooling device (24, 124, 256) is configured to generate an exothermic process by using the pump to compress gas into the first smaller chamber (90) from the second larger chamber (94), the thermal cooling device (24, 124, 256) is configured to generate an endothermic process by releasing the gas from the first smaller chamber (90) into the second larger chamber (94) by opening the third valve (98).

6. The biopsy device (20, 120, 220) according to any one of claims 1, 2, 3, and 5, comprising a first thermal sensor (76) coupled to the proximal end portion (50, 250) of the elongate biopsy needle (22, 222), wherein the first thermal sensor (76) is configured to sense the temperature of the proximal end portion (50, 250) of the elongate biopsy needle (22, 222), optionally comprising a second thermal sensor (78) coupled to the first thermo-mechanical coupler (70, 170), wherein the thermal sensor (78) is configured to sense the temperature of the first thermo-mechanical coupler (70, 170).

7. The biopsy device (20, 120, 220) according any one of claims 1 to 2, wherein the first thermo-mechanical coupler (70, 170) is an electro-mechanical switch, and the electro-mechanical switch is configured to open during a first period of engagement of the thermal cooling device (24, 124, 256) to thermally isolate the elongate biopsy needle (22, 222) from the thermal cooling device (24, 124, 256), optionally wherein the electro-mechanical switch is configured to move into a closed condition during a second period of engagement of the thermal cooling device (24, 124, 256), the closed condition completes a thermal connection between the thermal cooling device (24, 124, 256) and the first end (64, 264) of the temperature transfer member (62, 262).

8. A biopsy system (10, 110) comprising the biopsy device (20, 120, 220) according to claim 1, the elongate biopsy needle (22, 222) having a lumen, wherein the system (10, 110) further comprises a controller circuit (26) communicatively coupled to the thermal cooling device (24, 124, 256), wherein the controller circuit (26) is configured to execute program instructions to perform functions associated with a chilling effect at the distal end portion (54, 254) of the elongate biopsy needle (22, 222) generated by the thermal cooling device (24, 124, 256).

9. The biopsy system (10, 110) according to claim 8, the biopsy device (20, 120, 220) further comprising a driver assembly (46, 246) configured to move the elongate biopsy needle (22, 222), wherein the controller circuit (26) comprises a driver assembly circuit (40) and a thermal controller circuit (28), the driver assembly circuit (40) is communicatively coupled to the driver assembly (46, 246), the thermal controller circuit (28) is communicatively coupled to the thermal cooling device (24, 124, 256).

10. The biopsy system (10, 110) according to any one of claims 8 to 9, the controller circuit (26) is configured to selectively connect the first thermo-mechanical coupler (70, 170) to the first end (64, 264) of the temperature transfer member (62, 262).

11. The biopsy system (10, 110) according to any one of claims 8 to 10, wherein the first thermo-mechanical coupler (70, 170) is an electro-mechanical switch that is configured to selectively connect the thermal cooling device (24, 124, 256) to the first end (64, 264) of the temperature transfer member (62, 262), the thermal controller circuit (28) is configured to open the electro-mechanical switch during a first period of engagement, and the thermal cooling device (24, 124, 256) is configured to produce an exothermic effect during the first period of engagement.

12. The biopsy system (10, 110) according to any one of claims 8 to 11, wherein the first thermo-mechanical coupler (70, 170) is an electro-mechanical switch that is configured to selectively connect the thermal cooling device (24, 124, 256) to the first end (64, 264) of the temperature transfer member (62, 262), the thermal control circuit (28) is configured to close the electro-mechanical switch during a second period of engagement, and the thermal cooling device (24, 124, 256) is configured to produce an endothermic effect during the second period of engagement.

13. The biopsy system (10, 110) according to any one of claims 8 to 12, comprising a thermal sensor (76) coupled to the proximal end portion (50, 250) of the elongate biopsy needle (22, 222), wherein the controller circuit (26) includes a thermal controller circuit (28), the thermal controller circuit (28) comprises a processor circuit (30) and a memory circuit (32), the thermal controller circuit (28) is communicatively coupled to the thermal sensor (76), the thermal sensor (76) is configured to collect and deliver temperature datum to the thermal control circuit (28), the thermal controller circuit (28) is communicatively coupled to the thermal cooling device (24, 124, 256), the thermal controller circuit (28) is configured to compare the temperature datum to a threshold stored in the memory circuit (32), if the temperature datum is above the threshold then the thermal controller circuit (28) processes a thermal cooling instruction in the processor circuit (30) to selectively engage the thermal cooling device (24, 124, 256) to generate a chilling effect, if the thermal controller circuit (28) determines that the temperature datum is at or below the threshold then the thermal controller circuit (28) continues to sense all temperature data received from the thermal sensor (76) and the thermal cooling device (24, 124, 256) is not engaged.

14. The biopsy system (10, 110) according to any one of claims 8 to 13, wherein the biopsy system (10, 110) is configured to continuously operate the thermal cooling device (24, 124, 256) during a medical procedure.

15. The biopsy system (10, 110) according to any one of claims 8 to 14, wherein the thermal cooling device (24, 124, 256) comprises:
a dual chamber enclosure, the dual chamber enclosure comprises a first smaller chamber (90) having a first valve (92) and a second larger chamber (94) having a second valve (96), the first smaller chamber (90) and the second larger chamber (94) are fluidly connected through a third valve (98) configured to be sealed or opened; and
a pump coupled to each of the first valve (92) and the second valve (96),
wherein the thermal cooling device (24, 124, 256) is configured to generate an exothermic process by using the pump to compress gas into the first smaller chamber (90) from the second larger chamber (94), the thermal cooling device (24, 124, 256) is configured to generate an endothermic process by releasing the gas from the first smaller chamber (90) into the second larger chamber (94) by opening the third valve (98), the controller circuit (26) is communicatively coupled to the pump.

## Patentansprüche

1. Biopsievorrichtung (20, 120, 220), die eine zu kühlende Biopsienadel (22, 222) aufweist, umfassend:
eine längliche Biopsienadel (22, 222), die einen proximalen Endabschnitt (50, 250), einen Zwischenabschnitt (52, 252) und einen distalen Endabschnitt (54, 254) aufweist, wobei die längliche Biopsienadel (22, 222) aus einem thermisch leitfähigen Material gefertigt ist;
eine thermische Kühlvorrichtung (24, 124, 256);
ein Temperaturübertragungselement (62, 262), das sich zwischen dem proximalen Endabschnitt (50, 250) der länglichen Biopsienadel (22, 222) und der thermischen Kühlvorrichtung (24, 124, 256) erstreckt, wobei das Temperaturübertragungselement (62, 262) ein erstes Ende (64, 264) und ein zweites Ende (66, 266) aufweist;
einen ersten thermomechanischen Koppler (70, 170), der das erste Ende (64, 264) des Temperaturübertragungselements (62, 262) mit der thermischen Kühlvorrichtung (24, 124, 256) verbindet; und
einen zweiten thermomechanischen Koppler (72, 272), der das zweite Ende (66, 266) des Temperaturübertragungselements (62, 262) mit dem proximalen Endabschnitt (50, 250) der länglichen Biopsienadel (22, 222) verbindet.

2. Biopsievorrichtung (20, 120, 220) nach Anspruch 1, wobei die längliche Biopsienadel (22, 222) mindestens eines von einer länglichen Kanüle (226) und einem länglichen Mandrin (230) einschließt und das Temperaturübertragungselement (62, 262) ein starrer, länglicher, Vollzylinder ist, der aus Metall gebildet ist.

3. Biopsievorrichtung (20, 120, 220) nach einem der Ansprüche 1 bis 2, wobei die thermische Kühlvorrichtung (24, 124, 256) eine thermoelektrische Wärmepumpe ist.

4. Biopsievorrichtung (20, 120, 220) nach einem der Ansprüche 1 bis 2, wobei die thermische Kühlvorrichtung (24, 124, 256) ein Kanisterventil (274) und einen Umgebungsluftanschluss (276) umfasst, wobei die thermische Kühlvorrichtung (24, 124, 256) dazu konfiguriert ist, mit dem ersten thermomechanischen Koppler (70, 170) zu koppeln, und das Kanisterventil (274) dazu konfiguriert ist, einen Einwegkanister (280), der Druckgas enthält, aufzunehmen, wobei die thermische Kühlvorrichtung (24, 124, 256) dazu konfiguriert ist, das Kanisterventil (274) zu öffnen, um das Druckgas (280) durch den Umgebungsluftanschluss (276) aus dem Einwegkanister an die Umgebungsluft freizugeben, um einen Kühleffekt zu erzeugen, der von der thermischen Kühlvorrichtung (24, 124, 256) auf den ersten thermomechanischen Koppler (70, 170), auf das Temperaturübertragungselement (62, 262), auf den proximalen Endabschnitt (50, 250) und auf den distalen Endabschnitt (54, 254) der länglichen Biopsienadel (22, 222) übertragen wird.

5. Biopsievorrichtung (20, 120, 220) nach einem der Ansprüche 1 bis 2, wobei die thermische Kühlvorrichtung (24, 124, 256) umfasst:
ein Zweikammergehäuse, wobei das Zweikammergehäuse eine erste kleinere Kammer (90), die ein erstes Ventil (92) aufweist, und eine zweite größere Kammer (94), die ein zweitens Ventil (96) aufweist, umfasst, wobei die erste kleinere Kammer (90) und die zweite größere Kammer (94) über ein drittes Ventil (98), das dazu konfiguriert ist, abgedichtet oder geöffnet zu sein, fluidisch gekoppelt sind; und
eine Pumpe, die mit jedem von dem ersten Ventil (92) und dem zweiten Ventil (96) gekoppelt ist,
wobei die thermische Kühlvorrichtung (24, 124, 256) dazu konfiguriert ist, durch Verwenden der Pumpe, um Gas aus der zweiten größeren Kammer (94) in die erste kleinere Kammer (90) zu komprimieren, einen exothermen Prozess zu erzeugen, wobei die thermische Kühlvorrichtung (24, 124, 256) dazu konfiguriert ist, durch Freigeben des Gases aus der ersten kleineren Kammer (90) in die zweite größere Kammer (94) durch Öffnen des dritten Ventils (98) einen endothermen Prozess zu erzeugen.

6. Biopsievorrichtung (20, 120, 220) nach einem der Ansprüche 1, 2, 3, und 5, umfassend einen ersten Thermosensor (76), der mit dem proximalen Endabschnitt (50, 250) der länglichen Biopsienadel (22, 222) gekoppelt ist, wobei der erste Thermosensor (76) dazu konfiguriert ist, die Temperatur des proximalen Endabschnitts (50, 250) der länglichen Biopsienadel (22, 222) zu erfassen, optional umfassend einen zweiten Thermosensor(78), der mit dem ersten thermomechanischen Koppler (70, 170) gekoppelt ist, wobei der Thermosensor (78) dazu konfiguriert ist, die Temperatur des ersten thermomechanischen Kopplers (70, 170) zu erfassen.

7. Biopsievorrichtung (20, 120, 220) nach einem der Ansprüche 1 bis 2, wobei der erste thermomechanische Koppler (70, 170) ein elektromechanischer Schalter ist und der elektromechanische Schalter dazu konfiguriert ist, sich während einer ersten Einschaltdauer der thermischen Kühlvorrichtung (24, 124, 256) zu öffnen, um die längliche Biopsienadel (22, 222) thermisch von der thermischen Kühlvorrichtung (24, 124, 256) zu isolieren, optional wobei der elektromechanische Schalter dazu konfiguriert ist, sich während einer zweiten Einschaltdauer der thermischen Kühlvorrichtung (24, 124, 256) in einen geschlossenen Zustand zu bewegen, wobei der geschlossene Zustand eine thermische Verbindung zwischen der thermischen Kühlvorrichtung (24, 124, 256) und dem ersten Ende (64, 264) des Temperaturübertragungselements (62, 262) herstellt.

8. Biopsiesystem (10, 110), umfassend die Biopsievorrichtung (20, 120, 220) nach Anspruch 1, wobei die längliche Biopsienadel (22, 222) ein Lumen aufweist, wobei das System (10, 110) weiter eine Steuereinheitsschaltung (26) umfasst, die kommunikativ mit der thermischen Kühlvorrichtung (24, 124, 256) gekoppelt ist, wobei die Steuereinheitsschaltung (26) dazu konfiguriert ist, Programmanweisungen auszuführen, um Funktionen durchzuführen, die mit einem Kühleffekt am distalen Endabschnitt (54, 254) der länglichen Biopsienadel (22, 222) verknüpft sind, der von der thermischen Kühlvorrichtung (24, 124, 256) erzeugt wird.

9. Biopsiesystem (10, 110) nach Anspruch 8, die Biopsievorrichtung (20, 120, 220) weiter umfassend eine Treiberanordnung (46, 246), die dazu konfiguriert ist, die längliche Biopsienadel (22, 222) zu bewegen, wobei die Steuereinheitsschaltung (26) eine Treiberanordnungsschaltung (40) und eine thermische Steuereinheitsschaltung (28) umfasst,
wobei die Treiberanordnungsschaltung (40) kommunikativ mit der Treiberanordnung (46, 246) gekoppelt ist, die thermische Steuereinheitsschaltung (28) kommunikativ mit der thermischen Kühlvorrichtung (24, 124, 256) gekoppelt ist.

10. Biopsiesystem (10, 110) nach einem der Ansprüche 8 bis 9, wobei die Steuereinheitsschaltung (26) dazu konfiguriert ist, den ersten thermomechanischen Koppler (70, 170) selektiv mit dem ersten Ende (64, 264) des Temperaturübertragungselements (62, 262) zu verbinden.

11. Biopsiesystem (10, 110) nach einem der Ansprüche 8 bis 10, wobei der erste thermomechanische Koppler (70, 170) ein elektromechanischer Schalter ist, der dazu konfiguriert ist, die thermische Kühlvorrichtung (24, 124, 256) selektiv mit dem ersten Ende (64, 264) des Temperaturübertragungselements (62, 262) zu verbinden, wobei die thermische Steuereinheitsschaltung (28) dazu konfiguriert ist, den elektromechanischen Schalter während einer ersten Einschaltdauer zu öffnen, und die thermische Kühlvorrichtung (24, 124, 256) dazu konfiguriert ist, während der ersten Einschaltdauer einen exothermen Effekt zu produzieren.

12. Biopsiesystem (10, 110) nach einem der Ansprüche 8 bis 11, wobei der erste thermomechanische Koppler (70, 170) ein elektromechanischer Schalter ist, der dazu konfiguriert ist, die thermische Kühlvorrichtung (24, 124, 256) selektiv mit dem ersten Ende (64, 264) des Temperaturübertragungselements (62, 262) zu verbinden, wobei die thermische Steuerschaltung (28) dazu konfiguriert ist, den elektromechanischen Schalter während einer zweiten Einschaltdauer zu schließen, und die thermische Kühlvorrichtung (24, 124, 256) dazu konfiguriert ist, während der zweiten Einschaltdauer einen endothermen Effekt zu produzieren.

13. Biopsiesystem (10, 110) nach einem der Ansprüche 8 bis 12, umfassend einen Thermosensor (76), der mit dem proximalen Endabschnitt (50, 250) der länglichen Biopsienadel (22, 222) gekoppelt ist, wobei die Steuereinheitsschaltung (26) eine thermische Steuereinheitsschaltung (28) einschließt, wobei die thermische Steuereinheitsschaltung (28) eine Prozessorschaltung (30) und eine Speicherschaltung (32) umfasst, die thermische Steuereinheitsschaltung (28) kommunikativ mit dem Thermosensor (76) gekoppelt ist, der Thermosensor (76) dazu konfiguriert ist, ein Temperaturdatum zu sammeln und an die thermische Steuerschaltung (28) zu liefern, wobei die thermische Steuereinheitsschaltung (28) kommunikativ mit der thermischen Kühlvorrichtung (24, 124, 256) gekoppelt ist, die thermische Steuereinheitsschaltung (28) dazu konfiguriert ist, das Temperaturdatum mit einem in der Speicherschaltung (32) gespeicherten Schwellenwert zu vergleichen, wobei, wenn das Temperaturdatum über dem Schwellenwert liegt, die thermische Steuereinheitsschaltung (28) eine thermische Kühlanweisung in der Prozessorschaltung (30) verarbeitet, um die thermische Kühlvorrichtung (24, 124, 256) selektiv einzuschalten, um einen Kühleffekt zu erzeugen, wobei, wenn die Steuereinheitsschaltung (28) bestimmt, dass das Temperaturdatum auf oder unter dem Schwellenwert liegt, die thermische Steuereinheitsschaltung (28) dann fortfährt, alle vom Thermosensor (76) empfangenen Temperaturdaten zu erfassen, und die thermische Kühlvorrichtung (24, 124, 256) nicht eingeschaltet wird.

14. Biopsiesystem (10, 110) nach einem der Ansprüche 8 bis 13, wobei das Biopsiesystem (10, 110) dazu konfiguriert ist, die thermische Kühlvorrichtung (24, 124, 256) während einer medizinischen Prozedur kontinuierlich zu betreiben.

15. Biopsiesystem (10, 110) nach einem der Ansprüche 8 bis 14, wobei die thermische Kühlvorrichtung (24, 124, 256) umfasst:
ein Zweikammergehäuse, wobei das Zweikammergehäuse eine erste kleinere Kammer (90), die ein erstes Ventil (92) aufweist, und eine zweite größere Kammer (94), die ein zweitens Ventil (96) aufweist, umfasst, wobei die erste kleinere Kammer (90) und die zweite größere Kammer (94) über ein drittes Ventil (98), das dazu konfiguriert ist, abgedichtet oder geöffnet zu sein, fluidisch verbunden sind; und
eine Pumpe, die mit jedem von dem ersten Ventil (92) und dem zweiten Ventil (96) gekoppelt ist,
wobei die thermische Kühlvorrichtung (24, 124, 256) dazu konfiguriert ist, durch Verwenden der Pumpe, um Gas aus der zweiten größeren Kammer (94) in die erste kleinere Kammer (90) zu komprimieren, einen exothermen Prozess zu erzeugen, wobei die thermische Kühlvorrichtung (24, 124, 256) dazu konfiguriert ist, durch Freigeben des Gases aus der ersten kleineren Kammer (90) in die zweite größere Kammer (94) durch Öffnen des dritten Ventils (98) einen endothermen Prozess zu erzeugen, wobei die Steuereinheitsschaltung (26) kommunikativ mit der Pumpe gekoppelt ist.

## Revendications

1. Dispositif de biopsie (20, 120, 220) présentant une aiguille de biopsie (22, 222) à refroidir, comprenant :
une aiguille de biopsie (22, 222) allongée présentant une partie d'extrémité proximale (50, 250), une partie intermédiaire (52, 252), et une partie d'extrémité distale (54, 254), l'aiguille de biopsie (22, 222) allongée étant faite d'un matériau thermoconducteur ;
un dispositif de refroidissement thermique (24, 124, 256) ;
un élément de transfert de température (62, 262) qui s'étend entre la partie d'extrémité proximale (50, 250) de l'aiguille de biopsie (22, 222) allongée et le dispositif de refroidissement thermique (24, 124, 256), l'élément de transfert de température (62, 262) présentant une première extrémité (64, 264) et une seconde extrémité (66, 266) ;
un premier coupleur thermomécanique (70, 170) qui raccorde la première extrémité (64, 264) de l'élément de transfert de température (62, 262) au dispositif de refroidissement thermique (24, 124, 256) ; et
un second coupleur thermomécanique (72, 272) qui raccorde la seconde extrémité (66, 266) de l'élément de transfert de température (62, 262) à la partie d'extrémité proximale (50, 250) de l'aiguille de biopsie (22, 222) allongée.

2. Dispositif de biopsie (20, 120, 220) selon la revendication 1, dans lequel l'aiguille de biopsie (22, 222) allongée inclut au moins un ou une d'une canule (226) allongée et d'un stylet (230) allongé, et l'élément de transfert de température (62, 262) est un cylindre solide allongé et rigide constitué de métal.

3. Dispositif de biopsie (20, 120, 220) selon l'une quelconque des revendications 1 et 2, dans lequel le dispositif de refroidissement thermique (24, 124, 256) est une pompe à chaleur thermoélectrique.

4. Dispositif de biopsie (20, 120, 220) selon l'une quelconque des revendications 1 et 2, dans lequel le dispositif de refroidissement thermique (24, 124, 256) comprend une soupape de cartouche (274) et un orifice d'air ambiant (276), le dispositif de refroidissement thermique (24, 124, 256) étant configuré pour se coupler avec le premier coupleur thermomécanique (70, 170), et la soupape de cartouche (274) configurée pour recevoir une cartouche jetable (280) contenant du gaz comprimé, dans lequel le dispositif de refroidissement thermique (24, 124, 256) est configuré pour ouvrir la soupape de cartouche (274) afin de libérer le gaz comprimé de la soupape jetable (280) dans l'air ambiant à travers l'orifice d'air ambiant (276) pour générer un effet de refroidissement qui est transféré du dispositif de refroidissement thermique (24, 124, 256) au premier coupleur thermomécanique (70, 170), à l'élément de transfert de température (62, 262), à la partie d'extrémité proximale (50, 250), et à la partie d'extrémité distale (54, 254) de l'aiguille de biopsie (22, 222) allongée.

5. Dispositif de biopsie (20, 120, 220) selon l'une quelconque des revendications 1 et 2, dans lequel le dispositif de refroidissement thermique (24, 124, 256) comprend :
une enceinte à chambre double, l'enceinte à chambre double comprend une première chambre plus petite (90) présentant une première soupape (92) et une seconde chambre plus grande (94) présentant une deuxième soupape (96), la première chambre plus petite (90) et la seconde chambre plus grande (94) sont couplées fluidiquement à travers une troisième soupape (98) configurée pour être scellée ou ouverte ; et
une pompe couplée à chacune de la première soupape (92) et de la deuxième soupape (96),
dans lequel le dispositif de refroidissement thermique (24, 124, 256) est configuré pour générer un processus exothermique par l'utilisation de la pompe pour comprimer un gaz dans la première chambre plus petite (90) à partir de la seconde chambre plus grande (94), le dispositif de refroidissement thermique (24, 124, 256) est configuré pour générer un processus endothermique par la libération du gaz de la première chambre plus petite (90) dans la seconde chambre plus grande (94) par l'ouverture de la troisième soupape (98).

6. Dispositif de biopsie (20, 120, 220) selon l'une quelconque des revendications 1, 2, 3 et 5, comprenant un premier capteur thermique (76) couplé à la partie d'extrémité proximale (50, 250) de l'aiguille de biopsie (22, 222) allongée, dans lequel le premier capteur thermique (76) est configuré pour détecter la température de la partie d'extrémité proximale (50, 250) de l'aiguille de biopsie (22, 222) allongée, comprenant facultativement un second capteur thermique (78) couplé au premier coupleur thermomécanique (70, 170), dans lequel le capteur thermique (78) est configuré pour détecter la température du premier coupleur thermomécanique (70, 170).

7. Dispositif de biopsie (20, 120, 220) selon l'une quelconque des revendications 1 et 2, dans lequel le premier coupleur thermomécanique (70, 170) est un commutateur électromécanique, et le commutateur électromécanique est configuré pour s'ouvrir pendant une première période d'enclenchement du dispositif de refroidissement thermique (24, 124, 256) de manière à isoler thermiquement l'aiguille de biopsie (22, 222) allongée du dispositif de refroidissement thermique (24, 124, 256), facultativement, dans lequel le commutateur électromécanique est configuré pour se placer dans un état fermé pendant une seconde période d'enclenchement du dispositif de refroidissement thermique (24, 124, 256), l'état fermé réalisant un raccordement thermique entre le dispositif de refroidissement thermique (24, 124, 256) et la première extrémité (64, 264) de l'élément de transfert de température (62, 262).

8. Système de biopsie (10, 110) comprenant le dispositif de biopsie (20, 120, 220) selon la revendication 1, l'aiguille de biopsie (22, 222) allongée présentant une lumière, dans lequel le système (10, 110) comprend en outre un circuit de dispositif de commande (26) couplé de manière communicative au dispositif de refroidissement thermique (24, 124, 256), dans lequel le circuit de dispositif de commande (26) est configuré pour exécuter des instructions de programme de manière à remplir des fonctions associées à un effet de refroidissement au niveau de la partie d'extrémité distale (54, 254) de l'aiguille de biopsie (22, 222) allongée généré par le dispositif de refroidissement thermique (24, 124, 256).

9. Système de biopsie (10, 110) selon la revendication 8, le dispositif de biopsie (20, 120, 220) comprenant en outre un ensemble d'entraînement (46, 246) configuré pour déplacer l'aiguille de biopsie (22, 222) allongée, dans lequel le circuit de dispositif de commande (26) comprend un circuit d'ensemble d'entraînement (40) et un circuit de dispositif de commande thermique (28), le circuit d'ensemble d'entraînement (40) est couplé de manière communicative à l'ensemble d'entraînement (46, 246), le circuit de dispositif de commande thermique (28) est couplé de manière communicative au dispositif de refroidissement thermique (24, 124, 256).

10. Système de biopsie (10, 110) selon l'une quelconque des revendications 8 et 9, dans lequel le circuit de dispositif de commande (26) est configuré pour raccorder sélectivement le premier coupleur thermomécanique (70, 170) à la première extrémité (64, 264) de l'élément de transfert de température (62, 262).

11. Système de biopsie (10, 110) selon l'une quelconque des revendications 8 à 10, dans lequel le premier coupleur thermomécanique (70, 170) est un commutateur électromécanique qui est configuré pour raccorder sélectivement le dispositif de refroidissement thermique (24, 124, 256) à la première extrémité (64, 264) de l'élément de transfert de température (62, 262), le circuit de dispositif de commande thermique (28) est configuré pour ouvrir le commutateur électromécanique pendant une première période d'enclenchement, et le dispositif de refroidissement thermique (24, 124, 256) est configuré pour produire un effet exothermique pendant la première période d'enclenchement.

12. Système de biopsie (10, 110) selon l'une quelconque des revendications 8 à 11, dans lequel le premier coupleur thermomécanique (70, 170) est un commutateur électromécanique qui est configuré pour raccorder sélectivement le dispositif de refroidissement thermique (24, 124, 256) à la première extrémité (64, 264) de l'élément de transfert de température (62, 262), le circuit de dispositif de commande thermique (28) est configuré pour fermer le commutateur électromécanique pendant une seconde période d'enclenchement, et le dispositif de refroidissement thermique (24, 124, 256) est configuré pour produire un effet endothermique pendant la seconde période d'enclenchement.

13. Système de biopsie (10, 110) selon l'une quelconque des revendications 8 à 12, comprenant un capteur thermique (76) couplé à la partie d'extrémité proximale (50, 250) de l'aiguille de biopsie (22, 222) allongée, dans lequel le circuit de dispositif de commande (26) inclut un circuit de dispositif de commande thermique (28), le circuit de dispositif de commande thermique (28) comprend un circuit de processeur (30) et un circuit de mémoire (32), le circuit de dispositif de commande thermique (28) est couplé de manière communicative au capteur thermique (76), le capteur thermique (76) est configuré pour collecter et fournir une donnée de température au circuit de dispositif de commande thermique (28), le circuit de dispositif de commande thermique (28) est couplé de manière communicative au dispositif de refroidissement thermique (24, 124, 256), le circuit de dispositif de commande thermique (28) est configuré pour comparer la donnée de température à un seuil stocké dans le circuit de mémoire (32), si la donnée de température est supérieure au seuil, alors le circuit de dispositif de commande thermique (28) traite une instruction de refroidissement thermique dans le circuit de processeur (30) pour enclencher sélectivement le dispositif de refroidissement thermique (24, 124, 256) de manière à générer un effet de refroidissement, si le circuit de dispositif de commande thermique (28) détermine que la donnée de température est égale ou inférieure au seuil, alors le circuit de dispositif de commande thermique (28) continue à détecter toutes les données de température reçues en provenance du capteur thermique (76) et le dispositif de refroidissement thermique (24, 124, 256) n'est pas enclenché.

14. Système de biopsie (10, 110) selon l'une quelconque des revendications 8 à 13, dans lequel le système de biopsie (10, 110) est configuré pour faire fonctionner en continu le dispositif de refroidissement thermique (24, 124, 256) pendant une intervention médicale.

15. Système de biopsie (10, 110) selon l'une quelconque des revendications 8 à 14, dans lequel le dispositif de refroidissement thermique (24, 124, 256) comprend :
une enceinte à chambre double, l'enceinte à chambre double comprend une première chambre plus petite (90) présentant une première soupape (92) et une seconde chambre plus grande (94) présentant une deuxième soupape (96), la première chambre plus petite (90) et la seconde chambre plus grande (94) étant raccordées fluidiquement à travers une troisième soupape (98) configurée pour être scellée ou ouverte ; et
une pompe couplée à chacune de la première soupape (92) et de la deuxième soupape (96),
dans lequel le dispositif de refroidissement thermique (24, 124, 256) est configuré pour générer un processus exothermique par l'utilisation de la pompe pour comprimer un gaz dans la première chambre plus petite (90) à partir de la seconde chambre plus grande (94), le dispositif de refroidissement thermique (24, 124, 256) est configuré pour générer un processus endothermique par la libération du gaz de la première chambre plus petite (90) dans la seconde chambre plus grande (94) par l'ouverture de la troisième soupape (98), le circuit de dispositif de commande (26) est couplé de manière communicative à la pompe.
